# EUROPEAN PATENT SPECIFICATION

(11) **EP 1 901 787 B1**
(45) Date of publication and mention of the grant of the patent: **17.03.2010**
(21) Application number: 06762077.3
(22) Date of filing: 16.06.2006
(51) Int. Cl.: A61L 17/10, A61B 17/06

(54) **SURGICAL REPAIR PRODUCT BASED ON UHMWPE FILAMENTS**
CHIRURGISCHES REPARATURPRODUKT AUF BASIS VON UHMWPE-FÄDEN
PRODUIT DE RÉPARATION CHIRURGICALE À BASE DE FILAMENTS D'UHMWPE

(30) Priority: 05.07.2005 EP 05076531
(43) Date of publication of application: 26.03.2008
(73) Proprietor: DSM IP Assets B.V., 6411 TE Heerlen (NL)
(72) Inventor: MARISSEN, Roelof, NL-6121 HS Born (NL)
(74) Representative: Dorrestijn, Antoon
(86) International application number: PCT/EP2006/005848
(87) International publication number: WO 2007/003266

(56) References cited:
- EP-A- 0 002 090
- EP-A- 0 561 108
- EP-A- 1 293 218
- EP-A- 1 543 782
- WO-A-2005/066401
- US-A- 4 204 542

## Description

The invention relates to an elongated surgical repair product comprising a load-bearing member containing strands comprising a biocompatible high-performance polyethylene (HPPE) yarn substantially consisting of a plurality of ultra-high molar mass polyethylene (UHMWPE) filaments. The invention also relates to a method of making said product.

Such a product is known from EP 0561108 A2. In Example 6 of this publication a suture of spiroid braid construction was made from 15 strands each consisting of 215 denier Spectra^{®} 1000 yarn (having 60 filaments of about 3.9 dtex per filament). With an indicated knot pull strength (which is assumed to be the same as the knot tensile strength defined by the USP as being the tensile strength of a member with a simple knot tied in the middle of the member) of about 0.9 N/tex, such a braided member is one of the strongest sutures presently available.

In EP 12933218 A1 also such a product is disclosed. In Example 2 of this publication a braided member is described that is suitable for use as surgical suture or ligament, which member contains a core made from twisted 144 dtex HPPE yarns and a sheath braided from strands that consist of 100 dtex polyester yarn or 144 dtex HPPE yarn. The HPPE yarn used is indicated to be Spectra^{®} 2000, which yarn has (according to published data sheet information) a tensile strength of 3.4 N/tex and consists of 40 filaments of 3.6 dtex each. The knot tensile strength of this product is on the order of 0.5 N/tex.

An elongated surgical repair product is understood to be an article for use as for example a surgical suture for repairing soft body tissue, or as a cable, tape, ribbon or band for repairing or retaining body parts like bones; and the elongated product is of length dimension much larger than its cross-sectional dimensions (width, thickness). The repair product comprises at least one elongated member as load-bearing component, which is made from strands comprising the HPPE yarn; and may further comprise for example an anchor or a needle, a coating material, etc. Strands are the structural elements forming the member, and may contain one or more multi-filament yarns. A multi-filament yarn is a bundle of a plurality of continuous filaments, which may have been given a certain twist level to provide the yarn with some coherency.

Elongated surgical repair products like sutures have been made over time from a variety of materials for forming the strands, including flax, hair, cotton, silk, animal gut, and synthetic materials like polyesters, polyamides, and polyolefins like polyethylene or polypropylene. The material used may be absorbable or non-absorbable. Non-absorbable products are not dissolved or degraded by the body's natural action after implantation. Relevant material properties for use in sutures and other repair products include tensile strength, flexibility, elasticity, wettability, and other surface properties. A relatively new material for making non-absorbable surgical repair products, is multi-filament yarn made from ultra-high molar mass polyethylene (UHMWPE). The main advantages of this material include its biocompatibility, its good abrasion resistance, its flexibility, and especially its very high tensile strength. High-performance (or high-strength) polyethylene yarn is herein understood to be a multi-filament yarn having a tensile strength of more than 2.5 N/tex. Such yarn is called biocompatible if it fulfils the relevant requirements, of for example the FDA, with regard to other components being present in addition to the UHMWPE polymer (such as processing additives, solvent residues, and the like). Biocompatible HPPE yarn is therefore in the context of this patent application preferably to be understood a HPPE yarn, the UHMWPE filaments in the yarn containing less than 800 ppm of residual amounts of spin solvent. It is the high-performance polyethylene yarn that gives the member and the repair product its strength properties.

By indicating the knot tensile strength rather than the linear tensile strength to describe a load-bearing member for a surgical suture it is highlighted that while tensile strength is important, an applied knot substantially alters the strength: strength retention after a knot has been tied is often on the order of 40-50% of the linear strength. Although in applying a suture also other factors may play a role, knot tensile strength is considered a critical property, since surgeons appreciate to maximally tighten a knot by firmly pulling the ends; a.o. to better secure the knot and prevent slipping. Higher knot tensile strength further allows using thinner repair products, which is especially advantageous for minimal invasive surgical techniques. If a suture breaks during use, it breaks in virtually all cases at the knot. For thicker members that are to be used as artificial ligaments or tendons, i.e. as tension members, a high knot tensile strength may be even more relevant.

There is thus a constant need for a surgical repair product having improved strength, especially having higher knot tensile strength than presently available products.

The object of the invention is therefore to provide an elongated surgical repair product having such improved knot tensile strength, without sacrificing other favourable properties.

This object is achieved according to the invention with an elongated surgical repair product wherein the UHMWPE filaments have an effective diameter of at most 16 micrometer.

The elongated surgical repair product according to the invention shows higher knot tensile strength than known products, combined with good flexibility and handleability. A further advantage of the product according to the invention is that the strands (or yarns in a strand) may have a dimension or titer that can vary widely without deteriorating properties, meaning that relatively thick members can still contain a relatively low number of strands, and can be cost effectively made on existing machinery. A further advantage is that the member can be of various different constructions. Moreover, production of HPPE multifilament yarn of low titer is less efficient and economical than of higher titers.

Yet a further advantage is that the thin filaments show no or only a limited amount of loss in mechanical properties after cleaning to remove spin solvent, compared to thick filaments.

Although US 5456697 indicates a preference for using relatively thin filaments for making braided sutures, by mentioning a preferred filament titer range of 0.2-1.8 denier per filament; it is silent on possible effects on knot strength and on the use of high-performance polyethylene yarn.

The problem of improving knot tensile strength of a suture is also addressed in US 6712838 B2, but this patent publication provides a completely different solution by teaching a modified process for applying a coating to a braided member.

The product according to the invention comprises a member containing strands comprising high-performance polyethylene yarn substantially consisting of ultra-high molar mass polyethylene filaments.

HPPE multi-filament yarn can be made from UHMWPE polymer by a process generally referred to as gel spinning. Gel spinning of UHMWPE is well known to the person skilled in the art; and described in numerous publications, including EP 0205960 A, EP 0213208 A1, US 4413110, GB 2042414 A, EP 0200547 B1, EP 0472114 B1, WO 01/73173 A1, and Advanced Fiber Spinning Technology, Ed. T. Nakajima, Woodhead Publ. Ltd (1994), ISBN 1-855-73182-7. Gel spinning is understood to include at least the steps of spinning at least one filament from a solution of ultra-high molecular weight polyethylene in a spin solvent; cooling the filament obtained to form a gel filament; removing at least partly the spin solvent from the gel filament; and drawing the filament in at least one drawing step before, during or after removing spin solvent. Suitable spin solvents include for example paraffins, like paraffin oil and paraffin wax, mineral oil, kerosene, decalin, tetralin, toluene, lower n-alkanes, for example hexane, xylene, paraxylene, squalane, cyclo-octane. Parafin oil and decalin are most commonly used. Spin solvent may be removed by evaporation, by extraction, or by a combination of evaporation and extraction routes. Such HPPE yarns are commercially available as Spectra^{®} or Dyneema^{®} grades.

The yarn substantially consisting of a plurality of ultra-high molar mass polyethylene filaments indicates that there may be small amounts, e.g. at most 5 mass%, of other components present, like a coating or sizing.

Within the context of the present application UHMWPE is understood to be polyethylene with an intrinsic viscosity (IV, as determined according to method PTC-179 (Hercules Inc. Rev. Apr. 29, 1982) at 135°C in decalin, with dissolution time of 16 hours, with anti-oxidant DBPC in an amount of 2 g/l solution, and the viscosity at different concentrations extrapolated to zero concentration) of above 5 dl/g. Particularly suitable is UHMWPE with IV of between about 8 and 40 dl/g, more preferably between 10 and 30, or 12 and 28, or between 15 and 25 dl/g. These ranges represent an optimum in polymer processability and filaments properties. Intrinsic viscosity is a measure for molar mass (also called molecular weight) that can more easily be determined than actual molar mass parameters like Mn and Mw. There are several empirical relations between IV and Mw, but such relation is highly dependent on molar mass distribution. Based on the equation Mw = 5.37 x 10⁴ [IV]^{1.37} (see EP 0504954 A1) an IV of 8 dl/g would be equivalent to Mw of about 930 kg/mol.

Preferably, the UHMWPE is a linear polyethylene with less than one branch or side chain per 100 carbon atoms, and preferably less than one side chain per 300 carbon atoms, a branch usually containing at least 10 carbon atoms. The linear polyethylene may further contain up to 5 mol% of one or more comonomers, such as alkenes like propylene, butene, pentene, 4-methylpentene or octene.

In a preferred embodiment, the UHMWPE contains a small amount of relatively small groups as side chains, preferably a C1-C4 alkyl group. It is found that a filament from UHMWPE with a certain amount of such groups show reduced creep behaviour. Too large a side chain, or too high an amount of side chains, however, negatively affects the processing and especially the drawing behaviour of the filaments. For this reason, the UHMWPE preferably contains methyl or ethyl side chains, more preferably methyl side chains. The UHMWPE therefore contains preferably at least 0.2, 0.3, 0.4 or 0.5 methyl or ethyl side chains. The amount of side chains is preferably at most 20, more preferably at most 10 per 1000 carbon atoms.

The UHMWPE can be a single polymer grade, but also a mixture of two or more different grades, e.g. differing in IV or molar mass distribution, and/or number of side chains.

The UHMWPE filaments may further contain usual amounts, generally less than 5 mass% of customary additives, such as anti-oxidants, thermal stabilizers, colorants, nucleating agents, flow promoters, catalyst residues etc.; as long as these components are suitable for the use in a surgical product. The UHMWPE filaments preferably contain less than 800 ppm of residual amounts of spin solvent, more preferably less than 500, 250, or even less than 100 ppm, most preferably less than 50ppm. The filaments may also contain other polymers, preferably polyolefinic polymers, like other polyethylenes, polypropylenes, or their copolymers, including rubbery copolymers like EPDM, EPR, etc. The amount of such other polymer is always lower than the amount of UHMWPE in the filament, and is preferably not more than 30% of the UHMWPE filament.

The HPPE yarns are the components that contribute the most to the strength properties of the load-bearing member. The member may further comprise strands made from other materials, e.g. other biocompatible polymers, to provide some other additional properties to the member, including visual contrast. Suitable examples include non-absorbable materials like other polyolefins, or semi-aromatic polyesters like polyethylene terephthalate, or absorbable polymers like aliphatic polyesters based on e.g. lactides. Preferably, the member consists essentially of HPPE yarns for optimum strength performance. The load-bearing member may further comprise other components, for example compounds or coatings that provide some functional effect, like anti-microbial or anti-inflammatory effects.

The load-bearing member in the product according to the invention can be of various constructions. Suitable examples include knitted structures, various braided constructions, or combinations thereof. Preferably, the member is of a braided construction, which combines strength and flexibility, and is most commonly used for making surgical repair products. Suitable braided members include solid tubular or circular braids, spiroid braids, or flat braids if an oblong cross-section is preferred rather than a round member. It is also possible to apply so-called kern-mantle (or core-sheath), or braid-on-braid constructions as member, especially for heavier members of larger diameter. In a kern-mantle braid there is generally a core that is formed from twisted yarns surrounded by a braided cover or sheath, whereas a braid-on-braid has a braided core and sheath.

The strands, and the yarns, in the member in the product according to the invention normally run at a certain angle with the length direction of the member; that is at an angle with the longitudinal axis of the member, hereinafter referred to as strand angle, depending on the type of construction and the method of making. In a braided structure, the strands also cross each other, typically at an angle (also called braid angle) that is twice the strand angle. The strand angle is amongst others related to the level of twist in a twined or laid member, and to the braiding period (or pick count) in a braided member. Preferably, the strand angle in the load-bearing member in the product according to the invention is at most 30 degrees. This results in better strength efficiency, including knot strength efficiency. More preferably, the strand angle is at most 28, 26, 24, 22, or 20 degrees. The strand angle is preferably at least 5 degrees, to provide a certain level of coherence to the member. More preferably, the strand angle is at least 6, 7, 8, 9 or 10 degrees, since this tends to improve handleability and knotting behaviour of the member.

The elongated surgical repair product comprising a load-bearing member contains strands, and thus yarns, that can have a titer (or linear density) that may vary widely, for example from 5 to 3000 dtex; which is significantly wider than in prior art publications. For example, in the above cited US 5456697 publication it is clearly taught that preferably a yarn with a certain maximum number of filaments; that is a yarn with a yarn titer of at most about 90 denier (or about 100 dtex) should be used as strand in a braided suture member (see Table II therein). For making thicker or heavier members more strands should thus be used, requiring more complex braiding machines for manufacturing such members. In US5662682 it is indicated that in a spiroid braided suture an appropriate maximum yarn titer is about 300 denier. Preferably, the strand in the member according to the invention has a titer of at least 15 dtex, more preferably at least 25, 100, 250, 300, 350, or at least 400 dtex. A higher titer makes the production of the member less complex and more economical; especially for heavier members. The strand titer is preferably at most about 2750, 2500, 2250, 2000, 1800 dtex or even at most 1600 dtex to result in a more flexible member.

The strand in the member may consist of one multifilament yarn, but also of two or more yarns, which have been twisted or otherwise assembled together. The yarn preferably has a titer range as indicated above, both if the strand contains only one yarn, as in other cases. In preferred embodiments, the yarn has a titer in the range of 250-2000 dtex.

The size of the member in the product according to the invention can be in the full USP range for sutures (e.g. 12/0 to 2), and above. For application as for example orthopaedic cables, artificial tendon or ligament a member can have a (round) cross-section of up to about 5 mm. Expressed otherwise, suitable members can have a linear density or titer in the range of from 50 to 200000 dtex, preferably about 2000-100000 dtex.

The surgical repair product according to the invention comprises UHMWPE filaments that have an effective diameter of at most 16 micrometer. Within the context of the present application, the effective diameter is understood to be the averaged maximum cross-sectional dimension of the filaments. This effective diameter can be calculated by measuring the maximum dimension of the cross-section of at least 40 filaments on a SEM micrograph of a cross-section of a yarn (after embedding in e.g. epoxy resin). For virtually round filaments this effective diameter equals the diameter, for oblong or flattened filaments it is similar to the width. Preferably, the filaments have about a circular cross-section, because tensioned knots have complex stress fields with high stress gradients. If a cross section is non-circular, one cross-section dimension is larger than the other; such that locally the stress level may exceed tensile strength resulting in premature filament breakage. This stress gradient effect is minimized for round filaments. A filament of ultra-high molecular weight polyethylene, having a circular cross-section and an effective diameter of 16 micrometer has a titer of about 1.96 dtex.

The filaments in the member made in EP 0561108 A2 (Spectra® 1000) are virtually round and have a linear density of about 3.9 dtex, which means their average diameter is about 25 micrometer. The filaments applied in EP 12933218 A1 are of 3.6 dtex, which equals about 24 micrometer for a round cross-section.

Preferably, the effective diameter of the UHMWPE filaments in the product according to the invention is at most 15, more preferably at most 14, most preferably at most 13 micrometer. Advantages of such low diameter filaments include higher strength and higher flexibility of the resulting member, and more design freedom: yarns containing still more filaments and of higher titer can be used as strands to result in a member with advantageous performance.

For practical reasons, the filaments should be produceable in a stable spinning process, the UHMWPE filaments preferably have an effective diameter of at least about 4 micrometer (which equals about 0.1 dtex/filament for round cross-section), more preferably at least 5 micrometer.

The HPPE yarn in the member according to the invention may have a tenacity of at least 2.5 N/tex; preferably its tenacity is at least 2.8, 3.1, or even at least 3.4 N/tex. It is well known in the field of fibres and yarn technology that a multifilament yarn shows lower tenacity or tensile strength than the strength as measured on its constituent individual filaments. In general, the more filaments a yarn contains, the lower its tensile strength (breaking strength per unit of cross-sectional area, e.g. N/m² or Pa). It would thus be better to express the strength of a yarn, but also of a strand and a member, not as the ultimate force per cross-sectional area, but to apply a correction for increasing cross-section.

In a specifically preferred embodiment of the product according to the invention, the HPPE yarn is a multifilament yarn made from linear UHMWPE of IV 8-40 dl/g, containing n filaments and having a tensile strength of at least f*(n^{-0.065}) GPa, wherein factor f is at least 5.8 and n is at least 5. Such a very high-strength yarn can be made with a gel spinning process as described in detail in the copending application PCT/NL2004/000903.

The elongated surgical repair product according to the invention shows better strength after a knot has been tied in it than known products, preferably the product (and/or the member) has a knot tensile strength of at least 0.95 N/tex. Most preferably, the product has a knot tensile strength of at least 1.00, 1.05, or at least 1.1 N/tex.

The invention further relates to a kit for surgical repair, comprising an elongated member according to the invention. The kit may further comprise other auxiliary surgical tools and complementary instructions for use.

A further and surprising advantage of the invention is that the thin filaments, show no or only a limited amount of loss in mechanical properties after cleaning, compared to the thicker filaments. This is especially true in case of cleaning to very low levels of residual spin solvent by usual methods, for example by evaporation at elevated temperatures or by extraction.

Therefore the invention also relates to a yarn of ultra-high molecular weight polyethylene (UHMWPE), comprising a plurality of ultra-high molecular weight polyethylene (UHMWPE) filaments having an effective diameter of at most 16 micrometer, preferably at most 15 micrometer, more preferably at most 14 micrometer, most preferably at most 13 micrometer and having a spin solvent content of less than 500 ppm, preferably less than 250 ppm, more preferably less than 100 ppm, most preferably less than 50 ppm, the yarn having a tenacity of at least 3.6 N/tex, preferably at least 3.8 N/tex, more preferably 4.0 N/tex.

The invention further relates to a method of making an elongated surgical repair product according to the invention, comprising a step of assembling a plurality of strands comprising HPPE yarns, applying known methods. Suitable methods for assembling of strands include twisting, knitting, and braiding techniques. Typically, the minimum numbers of strands (or yarns) needed to make a member is 3, but the number of strands in the member is not specifically limited. It is an advantage of the present invention, that relatively heavy yarn and strands can be used, without properties of the member unacceptably deteriorating. This means that the total number of strands and yarns needed to make a heavy member can be lower than for known products, such that existing equipment, for example a 16-strand braiding machine, can be used.

Preferably, the invention relates to a method of making an elongated surgical repair product according to the invention by braiding a plurality of strands comprising HPPE yarns. Other preferred embodiments of the method of making an elongated surgical repair product according to the invention are analogous to the embodiments and preferred options as described above.

In a specifically preferred embodiment, the invention relates to a method of making an elongated surgical repair product comprising a load-bearing member, the method comprising a step of braiding a plurality of strands with a strand angle of from 5 to 30 degrees, which strands comprise a biocompatible high-performance polyethylene yarn having a titer of 250-2000 dtex and a tensile strength of at least 3.4 N/tex, which yarn substantially consists of a plurality of filaments made from linear ultra-high molar mass polyethylene of intrinsic viscosity 8-40 dl/g, and which filaments have an effective diameter of at most 16 micrometer.

More preferably, the method applies a HPPE yarn that contains n filaments and has a tensile strength of at least f*(n^{-0.065}) GPa, wherein factor f is at least 5.8 and n is at least 5.

The invention further relates to a method of repairing body tissue, wherein an elongated surgical repair product or a kit according to the invention is applied.

The invention will be further elucidated with reference to the following non-limiting examples.

### Example 1

An 8 mass% decalin solution was made of UHMWPE having about 0.3 methyl side-groups per 1000 C atoms and IV of 20 dl/g. This solution was spun using a spinplate containing 585 spinholes with an initial cylindrical channel of 3.5 mm diameter and UD of 18, followed by a conical contraction with cone angle 60° into a cylindrical channel of 0.8 mm diameter and UD of 10, into a nitrogen atmosphere applying a draw ratio in the air-gap of about 18. After quenching in a water bath the solidified filaments were drawn at a temperature profile of 110-140°C with a draw ratio of about 4. Two of these yarns were combined and subsequently drawn in an oven at about 151°C at a draw ratio of 6.5. Some relevant characteristics of the yarn thus obtained are given in Table 1. The filaments in the yarn have a virtually round cross-section.

8 strands of said yarn were braided into a member with about 4.3 picks per centimetre in a 2 over 2 construction. Tensile strength of the member was determined after tying a simple knot at about half the length. A simple knot is also referred to as single knot or overhand knot in literature. The strand angle was measured manually on micrographs made of a tensioned member. Methods used:
- IV: the Intrinsic Viscosity is determined according to method PTC-179 (Hercules Inc. Rev. Apr. 29, 1982) at 135°C in decalin, the dissolution time being 16 hours, with DBPC as anti-oxidant in an amount of 2 g/l solution, by extrapolating the viscosity as measured at different concentrations to zero concentration;
- Side chains: the number of side chains in a UHMWPE sample is determined by FTIR on a 2 mm thick compression moulded film, by quantifying the absorption at 1375 cm ⁻¹ using a calibration curve based on NMR measurements (as in e.g. EP 0269151);
- Effective filament diameter: a small length of yarn is embedded in epoxy resin. The cross-section is examined with SEM on microtomed samples, and the maximum cross-sectional dimension of at least 40 filaments is measured on a micrograph and averaged;
- Tensile properties: tensile strength (or strength), tensile modulus (or modulus) and elongation at break (or eab) are defined and determined on multifilament yarns with a procedure in accordance with ASTM D885M, using a nominal gauge length of the fibre of 500 mm, a crosshead speed of 50%/min and Instron 2714 clamps, of type Fibre Grip D5618C. On the basis of the measured stress-strain curve the modulus is determined as the gradient between 0.3 and 1% strain. For calculation of the modulus and strength, the tensile forces measured are divided by the titre, as determined by weighing 10 metres of fibre; values in GPa are calculated assuming a density of 0.97 g/cm³. Strength of braided members was measured (after tying one simple knot) on a Zwick 1435 apparatus with Instron 1497K clamps.
- Strand angle: with an optical microscope a photo is made of the braided member while keeping it under elongational tension; the angle the strands make with the longitudinal axis is measured on a micrograph (average of at least 5 measurements).

### Example 2

Example 1 was repeated, but the member was now braided with 6 picks per centimetre; resulting in a higher strand angle and slightly lower knot tensile strength.

### Example 3.

Further increasing the tightness of the braid structure, that is applying about 8.5 picks per centimetre, resulted in a member with strand angle of 35 degrees. Despite the high strand angle a relatively favourable knot strength was obtained.

### Examples 4-6.

Analogously to Example 1 yarn was spun, but applying a lower final draw ratio: after assembling of two intermediate yarn products and the final drawing step with draw ratio 5.7 a yarn of somewhat higher titer (and slightly thicker filaments), and somewhat lower tensile strength resulted; see table 1. This yarn was used to make three 8x1 braided members, with 2.1, 3.6, and 6 picks per centimetre, respectively. All braided members showed a strand angle within 5-30 degrees, and knot tensile strength of above 0.90 N/tex.

### Comparative Experiment A.

A commercial orthopaedic composite suture, marketed by Arthrex as FiberWire^{®} #2, shows a knot strength of 5.44 kg. This suture of about 2700 dtex is composed of a core formed from 3 Spectra^{®} 2000 144 dtex UHMWPE yarns, surrounded by a 16-strand braided cover containing 8 165 dtex Spectra^{®} yarns and 8 120 dtex polyester yarns. Knot tensile strength of this product was found to be about 0.5 N/tex.

### Comparative Experiments B and C.

An 8-strand braid was made in a 1 over 1 construction with 2.1 picks per centimeter from Dyneema^{®} SK60 1760 dtex yarn.

An 8-strand braid was made in a 2 over 2 construction with 6 picks per centimeter from Dyneema^{®} SK65 440 dtex yarn.

The filaments in these yarns have an oblong cross-section of average width about 23 micron.

**Table 1**

| *Experiment* | *Yarn characteristics* | | | *Member characteristics* | | |
|---|---|---|---|---|---|---|
| | *Titer* | *Tenacity* | *Filament diameter* | *Strand angle* | *Titer* | *Knot tensile strength* |
| | (dtex) | (N/tex) | (micrometer) | (degrees) | (dtex) | (N/tex) |
| Ex.1 | 928 | 4.1 | 11 | 20 | 7750 | 1.11 |
| Ex. 2 | 928 | 4.1 | 11 | 22 | 8080 | 1.09 |
| Ex.3 | 928 | 4.1 | 11 | 35 | 8850 | 0.89 |
| Ex. 4 | 1054 | 3.9 | 12 | 10 | 8590 | 1.18 |
| Ex. 5 | 1054 | 3.9 | 12 | 15 | 8880 | 1.05 |
| Ex. 6 | 1054 | 3.9 | 12 | 26 | 9270 | 0.94 |
| Comp. exp. A | 144/165 | 3.4 | 24 | 23 | 2700 | 0.5 |
| Comp. exp. B | 1745 | 2.9 | 23 | 17 | 14570 | 0.73 |
| Comp. exp. C | 441 | 3.1 | 23 | 20 | 3710 | 0.89 |

## Claims

1. Elongated surgical repair product comprising a load-bearing member containing strands comprising a biocompatible high-performance polyethylene (HPPE) yarn substantially consisting of a plurality of ultra-high molar mass polyethylene (UHMWPE) filaments, **characterized in that** the UHMWPE filaments have an effective diameter of at most 16 micrometer.

2. Product according to claim 1, wherein the member is of a braided construction.

3. Product according to claim 1 or 2, wherein the strands in the load-bearing member run at a strand angle of at most 30 degrees.

4. Product according to any one of claims 1-3, wherein the strand angle is 8-26 degrees.

5. Product according to any one of claims 1-4, wherein the strand has a titer of 25 - 2500 dtex.

6. Product according to any one of claims 1-4, wherein the strand has a titer of 250 - 2000 dtex.

7. Product according to any one of claims 1-6, wherein the filaments have an effective diameter of at most 14 micrometer.

8. Product according to any one of claims 1-7, wherein the product has a knot tensile strength of at least 1.00 N/tex.

9. Method of making an elongated surgical repair product comprising a load-bearing member, the method comprising a step of braiding a plurality of strands with a strand angle of from 5 to 30 degrees, which strands comprise a biocompatible high-performance polyethylene yarn having a titer of 250-2000 dtex and a tensile strength of at least 3.4 N/tex, which yarn substantially consists of a plurality of filaments made from linear ultra-high molar mass polyethylene of intrinsic viscosity 8-40 dl/g, and which filaments have an effective diameter of at most 16 micrometer.

10. A yarn of ultra-high molecular weight polyethylene (UHMWPE), comprising a plurality of ultra-high molecular weight polyethylene (UHMWPE) filaments having an effective diameter of at most 16 micrometer and having a spin solvent content of less than 500 ppm, the yarn having a tenacity of at least 3.6 N/tex.

## Patentansprüche

1. Längliches Produkt für die chirurgische Reparation, umfassend ein tragendes Glied, enthaltend Stränge, umfassend ein biokompatibles Hochleistungspolyethylengarn (HPPE), im wesentlichen bestehend aus mehreren Filamenten aus Polyethylen ultrahoher Molmasse (UHMWPE), **dadurch gekennzeichnet, daß** die UHMWPE-Filamente einen effektiven Durchmesser von höchstens 16 Mikrometer aufweisen.

2. Produkt nach Anspruch 1, bei dem es sich bei dem Glied um ein Geflecht handelt.

3. Produkt nach Anspruch 1 oder 2, bei dem die Stränge in dem tragenden Glied unter einem Strangwinkel von höchstens 30 Grad verlaufen.

4. Produkt nach einem der Ansprüche 1-3, bei dem der Strangwinkel 8-26 Grad beträgt.

5. Produkt nach einem der Ansprüche 1-4, bei dem der Strang einen Titer von 25-2500 dtex aufweist.

6. Produkt nach einem der Ansprüche 1-4, bei dem der Strang einen Titer von 250-2000 dtex aufweist.

7. Produkt nach einem der Ansprüche 1-6, bei dem die Filamente einen effektiven Durchmesser von höchstens 14 Mikrometer aufweisen.

8. Produkt nach einem der Ansprüche 1-7, bei dem das Produkt eine Knotenzugfestigkeit von mindestens 1,00 N/tex aufweist.

9. Verfahren zur Herstellung eines länglichen Produkts für die chirurgische Reparation, umfassend ein tragendes Glied, bei dem man mehrere Stränge mit einem Strangwinkel von 5-30 Grad flicht, die ein biokompatibles Hochleistungspolyethylengarn mit einem Titer von 250-2000 dtex und einer Zugfestigkeit von mindestens 3,4 N/tex umfassen, das im wesentlichen aus mehreren Filamenten aus linearem Polyethylen ultrahoher Molmasse mit inhärenter Viskosität 8-40 dl/g besteht, die einen effektiven Durchmesser von höchstens 16 Mikrometer aufweisen.

10. Garn aus ultrahochmolekularem Polyethylen (UHMWPE), umfassend mehrere Filamente aus ultrahochmolekularem Polyethylen (UHMWPE) mit einem effektiven Durchmesser von höchstens 16 Mikrometer und mit einem Spinnlösungsmittelgehalt von weniger als 500 ppm, wobei das Garn eine Feinheitsfestigkeit von mindestens 3,6 N/tex aufweist.

## Revendications

1. Produit de réparation chirurgicale allongé comprenant un élément de support de charge qui contient des brins qui comprennent un fil de polyéthylène haute performance (HPPE) biocompatible constitué essentiellement d'une pluralité de filaments de polyéthylène de masse moléculaire très élevée (UHMWPE), **caractérisé en ce que** les filaments UHMPWE présentent un diamètre effectif d'au plus seize micromètres.

2. Produit selon la revendication 1, dans lequel l'élément est d'une construction tressée.

3. Produit selon la revendication 1 ou 2, dans lequel les brins dans l'élément de support de charge se trouvent à un angle de brin d'au plus 30 degrés.

4. Produit selon l'une quelconque des revendications 1 à 3, dans lequel l'angle du brin varie de 8 à 26 degrés.

5. Produit selon l'une quelconque des revendications 1 à 4, dans lequel le brin possède un titre de 25 à 2500 dtex.

6. Produit selon l'une quelconque des revendications 1 à 4, dans lequel le brin possède un titre de 250 à 2000 dtex.

7. Produit selon l'une quelconque des revendications 1 à 6, dans lequel les filaments possèdent un diamètre effectif d'au plus quatorze micromètres.

8. Produit selon l'une quelconque des revendications 1 à 7, dans lequel le produit possède une résistance à la traction au noeud d'au moins 1,00 N/tex.

9. Procédé de fabrication d'un produit de réparation chirurgicale allongé comprenant un élément de support de charge, le procédé comprenant une étape de tressage d'une pluralité de brins dont l'angle de brin varie de 5 à 30 degrés, lesquels brins comprennent un fil de polyéthylène haute performance biocompatible dont le titre varie de 250 à 2000 dtex et dont la résistance à la traction est d'au moins 3,4 N/tex, lequel fil est constitué essentiellement d'une pluralité de filaments constitués de polyéthylène à masse moléculaire linéaire très élevée d'une viscosité intrinsèque de 8 à 40 dl/g, et lesquels filaments présentent un diamètre effectif d'au plus seize micromètres.

10. Fil d'un polyéthylène à poids moléculaire très élevée (UHMWPE), comprenant une pluralité de filaments de polyéthylène à poids moléculaire très élevée (UHMWPE) dont le diamètre effectif est d'au plus seize micromètres et dont la teneur en solvant spin est inférieure à 500 ppm, le fil ayant une ténacité d'au moins 3,6 N/tex.
